# EUROPEAN PATENT APPLICATION

(11) **EP 2 796 855 A1**
(43) Date of publication of application: **29.10.2014**
(21) Application number: 14165427.7
(22) Date of filing: 22.04.2014
(51) Int. Cl.: G01N 15/14

(54) **Particle analysis apparatus and production method thereof**

(30) Priority: 22.04.2013 JP 2013089380
(71) Applicant: Horiba, Ltd., Kyoto-city, Kyoto 601-8510 (JP)
(72) Inventor: Miyamura, Kazuhiro, Kyoto, 601-8510 (JP); Ukon, Juichiroh, Kyoto, 601-8510 (JP)
(74) Representative: Isarpatent

(57) **Abstract**

An apparatus including a flow cell part 1 having a flow channel 11 where the sample solution containing the particles to be analyzed flows; a light irradiation device 2 for irradiating an irradiation light L1 as a beam light L2 to a light irradiation point in the flow channel; and a light receiving device for detecting the light L3 resulting from the irradiation of the beam light L1 to the light irradiation point. The light irradiation device 2 has a light source device Ls and a beam-light-forming-film 21 composed of an opaque film 22 and a light transmitting hole 23 formed in the film 22. Using the light transmitting hole 23, the cross sectional shape of the irradiation light L1 is changed to that of the beam light L2, whereby optical apparatuses such as lens and the like on the irradiation side are omitted.

## Description

### FIELD OF THE INVENTION

The present invention relates to a particle analysis apparatus for analyzing particles in a liquid, such as blood cells and the like, based on at least flow cytometry, and a production method thereof.

### BACKGROUND OF THE INVENTION

As a method for optically analyzing blood cells in the blood such as red blood cell, leukocyte, platelet and the like, and counting same and the like, flow cytometry is known. Flow cytometry is a technique including irradiating a predetermined irradiation light as a beam light focused on blood cells in a sample solution (sample liquid) advancing through a flow channel, and distinguishing the blood cells from the resulting optical characteristics such as light scattering, light absorbance and the like (e.g., JP-A-H8(1996)-327529).

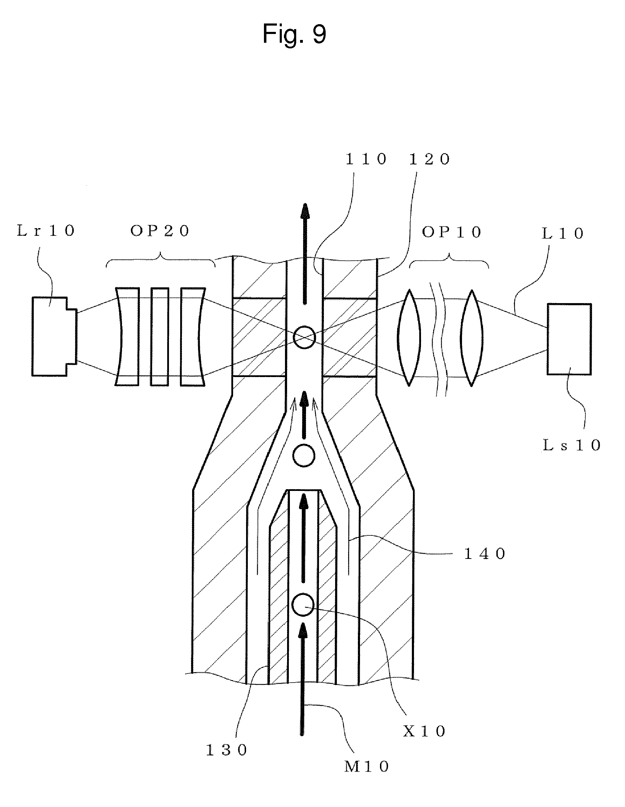

Fig. 9 is a sectional view of a configuration example of an apparatus for flow cytometry. An apparatus shown in this Figure is also called a flow cytometer.

In flow cytometry, as shown in this Figure, a sample solution M10 containing blood cells flows through a flow channel 110. An irradiation light L10 is irradiated from a light source device Ls10 through an optical system OP10 on a light irradiation point in the flow channel. Then, various optical characteristics such as the level of scattering produced when the irradiation light L10 hits blood cells X10, the level of light absorption by each blood cell and the like are measured by a light receiving device Lr10 via an optical system OP20, and the size, kind, state and the like of the blood cells are identified from the measurement results.

A measurement apparatus 120 containing the aforementioned flow channel 110 is also called a flow cell.

At least a part of the channel wall of a flow cell 120 is transparent so that the irradiation light L10 can be irradiated in the flow channel 110.

In the apparatus configuration of Fig. 9, the upstream side of the flow channel 110 (lower side of light irradiation point in the Figure) has a double tube structure, wherein the flow of the sample solution M10 from an inner tube 130 is surrounded by a sheath flow 140 from the outer tube and enters into the flow channel 110. Due to this configuration, the flow of the sample solution becomes narrow and the blood cells pass through the flow channel 110 one by one in an orderly manner. As a result, the irradiation light L10 can be applied (irradiated) on the individual blood cells.

However, in the configuration of conventional apparatuses as shown in Fig. 9, an irradiation light L10 is irradiated on a point in the center of a narrow flow in the flow channel 110, and therefore, many optical system parts OP10 such as lenses and the like need to be arranged. Therefore, down-sizing of the apparatus becomes difficult for a configuration of such a large-scale optical system.

Moreover, it is necessary to adjust such optical system parts with a high degree of accuracy as to the irradiation position and focus, and the cost of assembly becomes high.

Furthermore, when a blood cell analysis apparatus is a palm-top type (hand-sized type), a flow cell part in a preferable embodiment is a replaceable compact cartridge that can be disposed of after each measurement. However, the cartridge needs to be produced with a high degree of accuracy so that the adjustment of the above-mentioned optical system will not be necessary (particularly, the position of the irradiation point will not move toward 3-dimensinal directions) every time the cartridge is replaced, which increases the cost of the cartridge.

In addition, in recent years, an apparatus wherein the above-mentioned flow cytometer is further added with a configuration to perform an electrical resistance method (also called an impedance method) has been developed.

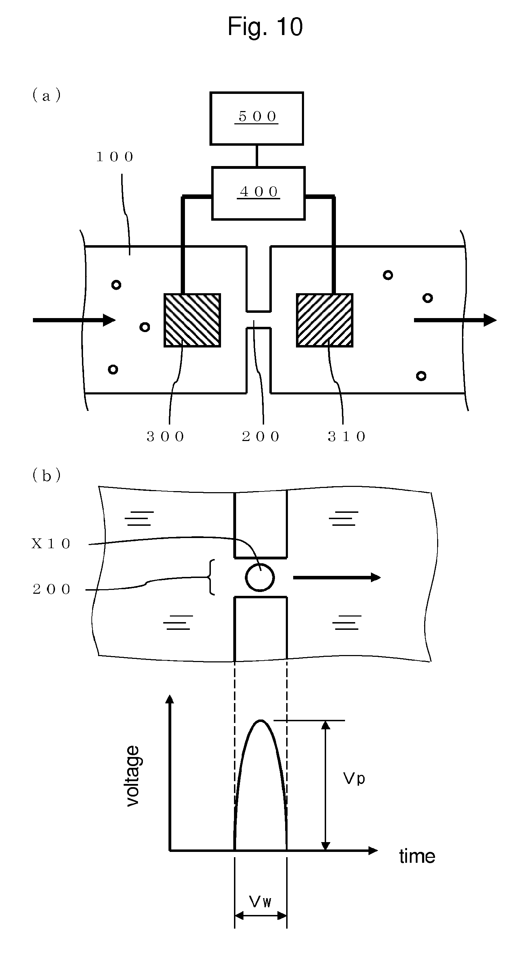

The electrical resistance method is a technique including, as shown in Fig. 10(a), introducing a sample solution M10 obtained by dispersing a given sample blood in a diluting liquid into a flow channel 100, setting an aperture (small through-hole) 200 having a smaller cross-sectional area of the flow channel like an orifice in the flow channel 100, providing a pair of electrodes 300, 310 at positions having the aperture 200 in between, and measuring the volume of the particles passing through the aperture 200 based on the changes of the electrical characteristics between the electrodes.

When one blood cell X10 passes through the aperture 200, the electrical resistance or impedance between the electrodes varies in a pulse-shape. Thus, when a voltage is being applied to the pair of electrodes 300, 310 from a constant-current power supply 400, the applied voltage also varies in a pulse-shape (a pulse voltage), as shown in Fig. 10(b), according to the above-mentioned impedance variation.

By enumerating (counting) the number of pulse voltages by, for example, a computing part 500 or the like connected to the constant-current power supply, it is possible to know the number of blood cells.

Also, since the pulse voltage height Vp on passage of a blood cell through the aperture is proportional to the size of the blood cell, the large or small volume of the blood cell can be determined from the pulse voltage height. When the flow velocity is constant, the pulse voltage width Vw increases in proportion to the size of the particle, and therefore, the large or small volume of the particle can also be determined to some extent from the pulse voltage width Vw.

The configuration of an apparatus for counting blood cells by the electrical resistance method is described in detail in, for example, JP-A-2004-257768, JP-A-2011-180117, JP-A-2005-062137 and the like. In the apparatus of JP-A-2011-180117, the flow channel on the downstream side of the aperture diverges in a unique configuration and, in the apparatus of JP-A-2005-062137, a pair of electrodes is set on the downstream side of the aperture in a unique configuration. The basic principle of the electrical resistance method, wherein an aperture is positioned between a pair of electrodes and the size of the particles is determined, is the same as that mentioned above.

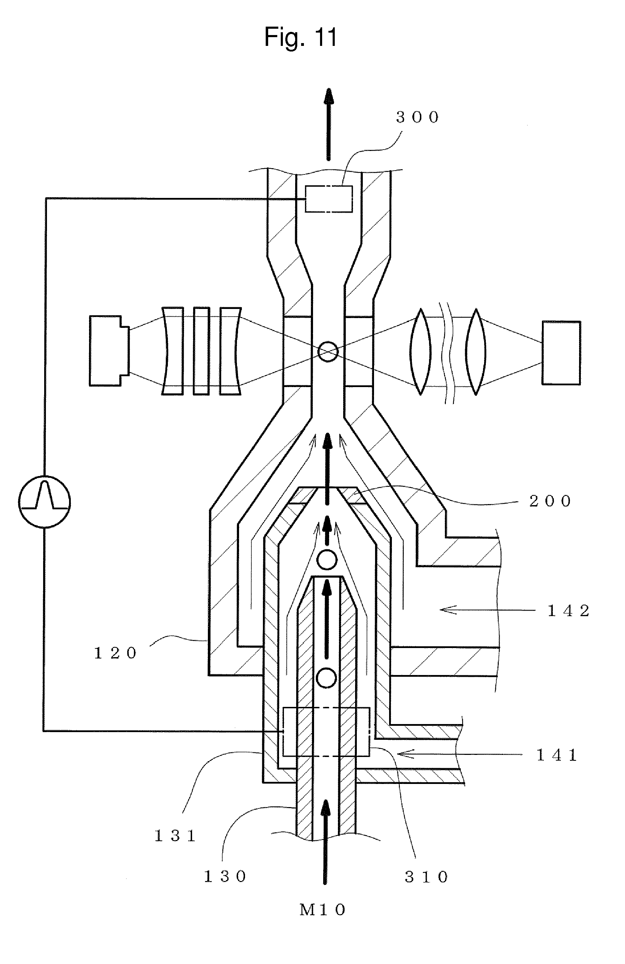

Fig. 11 is a sectional view of the configuration wherein an aperture and an electrode pair for the electrical resistance method are incorporated into the flow cytometer of Fig. 9. The flow of the sample solution, formation of the sheath flow, and the whole configuration of the optical system for the light irradiation are similar to those in Fig. 9. The symbol of each part of the optical system is the same as that in Fig. 9 and omitted.

In the configuration of the apparatus of Fig. 11, in a flow channel heading to the top of the Figure from the downside thereof, an aperture 200 is set immediately before the irradiation point of the beam light, a pair of electrodes (shown with a dashed line) 300, 310 is provided such that the aperture is interposed between them in the flow direction, and the electrical resistance method can be performed.

In the apparatus of Fig. 11, the upstream side of the flow channel (downside of the Figure) has a triple pipe structure, wherein the flow of the sample solution M10 from an inner tube 130 is surrounded by the first sheath flow 141 in a middle tube 131, enters as a narrow flow into an aperture 200, and the flow after passing through the aperture is surrounded by the second sheath flow 142 to become a flow with a suppressed turbulence and enters into the irradiation point.

An apparatus wherein parts for the electrical resistance method are incorporated into a flow cytometer, as shown in Fig. 11, has not only the above-mentioned problem relating to optical system parts (the problem of large-scale structure), but also the following problem of a time lag between two kinds of measurements.

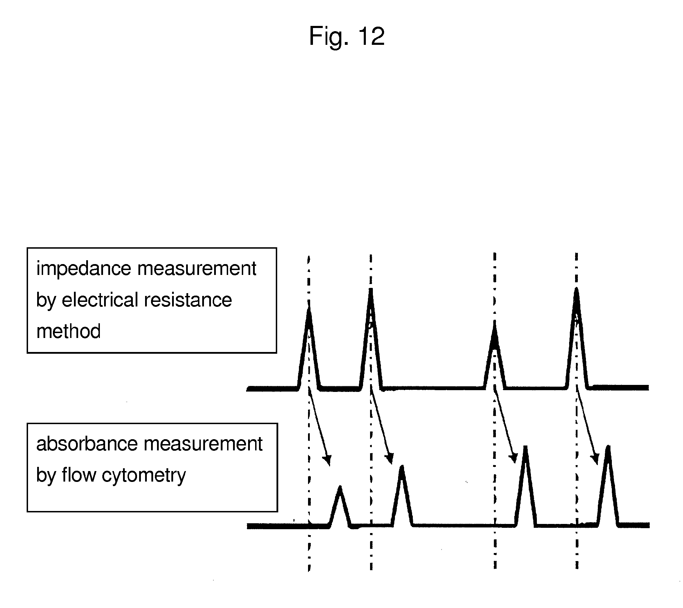

That is, in the configuration of Fig. 11, an impedance measurement (electrical resistance method) is performed when a certain, one particle passes through an aperture 200, and then, the measurement of light absorbance and the like (flow cytometry) is performed when the particle passes the irradiation point. As shown in Fig. 12, therefore, a time lag (temporal deviation) occurs in the measurement results of one particle by these two measurement methods.

Therefore, it is necessary to correct the time lag by a data processing software and the like, and relate these two kinds of measurement results as data pairs (data pair of [particle volume, light absorbance] and the like) relating to one particle. However, since such time lag varies depending on the flow rate of individual apparatuses, the shape of the flow cell, manufacturing error and the like, not only the correction using a data processing software but also accuracy of the parts and fine adjustment are also necessary to make the flow rate of a sample solution and the flow rate of a sheath liquid fall within the predetermined ranges.

The problems relating to the optical system parts in the above-mentioned flow cytometry, and the problem of time lag when the electrical resistance method is applied also occur not only for the counting apparatus and blood cell classifying device targeting the blood cells, but also for an apparatus for performing the analysis of various particles by flow cytometry.

The problem of the present invention is to solve the above-mentioned problems associated with an apparatus for flow cytometry, and further provide a particle analysis apparatus capable of solving the above-mentioned problem of time lag caused by a configuration for performing the electrical resistance method, and a production method thereof.

### SUMMARY OF THE INVENTION

The main configuration of the present invention is as follows.
(1) A particle analysis apparatus having a configuration adapted to analyze particles in a sample solution at least by flow cytometry, the apparatus comprising:
   a flow cell part having a flow channel where the sample solution containing the particles to be analyzed flows;
   a light irradiation device for irradiating an irradiation light as a beam light to a light irradiation point in the flow channel; and
   a light receiving device for detecting the light resulting from the irradiation of the beam light to the light irradiation point;
      wherein
      said light irradiation device comprises:
      a light source device for emitting the irradiation light; and
      a beam-light-forming-film composed of an opaque film prohibiting transmission of the irradiation light, and a light transmission through hole capable of changing the cross sectional shape of the irradiation light to a cross sectional shape of the beam light,
      and wherein
      the light irradiated from the light source device passes through the light transmitting hole in the beam-light-forming-film to form the beam light to be irradiated on the light irradiation point.
(2) The particle analysis apparatus of the above-mentioned (1), wherein the flow cell part and the flow channel are configured by a groove formed on a first surface, which is one main surface of the substrate for forming the flow channel, and a covering layer laminated on said substrate to cover and provide the groove as a conduit flow channel, wherein
   the covering layer is transparent, and the above-mentioned beam-light-forming-film is further laminated thereon, or the covering layer is also the above-mentioned beam-light-forming-film.
(3) The particle analysis apparatus of the above-mentioned (1) or (2), further comprising a second surface, which is the other main surface of the substrate for forming the flow channel, integrally formed with a curved surface part that functions as a lens for condensing the light, that has passed the light irradiation point, toward the light receiving device, said curved surface part having, as the center, a region corresponding to the light irradiation point in the flow channel in the first surface.
(4) The particle analysis apparatus of any of the above-mentioned (1)-(3), having a configuration also enabling analysis of particles in a sample solution by an electrical resistance method, wherein
   the above-mentioned flow channel comprises a micro zone containing the light irradiation point and having a decreased cross-sectional area such that the micro zone becomes an aperture for the electrical resistance method, and electrodes for performing the electrical resistance method, which are provided at positions placing the aperture between them, which apparatus enabling the flow cytometry and the electrical resistance method to be performed in a single micro zone.
(5) The particle analysis apparatus of any of the above-mentioned (1)-(4), wherein the flow channel diverges in the above-mentioned micro zone or at an outlet port on the downstream side of the micro zone.
(6) A method of producing the particle analysis apparatus of any of the above-mentioned (1)-(5), comprising the steps of:
   fixing an opaque film to the flow cell part having the flow channel so as to cover a region including the flow channel containing at least the light irradiation point, and
   processing the opaque film to form a light transmitting hole at a position corresponding to the light irradiation point to provide a beam-light-forming-film.

### BRIEF DESCRIPTION OF THE DRAWINGS

Fig. 1 is a partial sectional view schematically showing one example of the particle analysis apparatus of the present invention, which is cut along the flow channel to reveal the inside structure. In this Figure, only the part from a light source device to a light emitting element, in which the light irradiation point is the center, is illustrated, and the structure of the preceding stage and the like for forming the sheath flow shown in Fig. 9 and Fig. 11 are omitted.
Fig. 2 is a partial sectional view showing a preferable configuration example of a flow cell part suitable for using a beam-light-forming-film in the present invention, wherein a light source device and a light receiving device are omitted. In Fig. 2(a), the apparatus is cut along the flow direction of the flow channel. Fig. 2 (b) is a cross-section along the A-A line of Fig. 2(a), which is viewed from the arrow direction.
Fig. 3 is a sectional view explaining the characteristic step in the production method of the particle analysis apparatus of the present invention.
Fig. 4 is a sectional view of a configuration example wherein the lens for light reception is integrally formed in the present invention.
Fig. 5 is a sectional view of a configuration example of light irradiation in the present invention for flow cytometry in the aperture for the electrical resistance method, wherein a light source device and a light receiving device are omitted. In Fig. 5(a), the apparatus is cut along the flow direction of the flow channel. Fig. 5(b) is a top view of Fig. 5(a), wherein the outline of the main part hidden by a beam-light-forming-film is shown with a broken line.

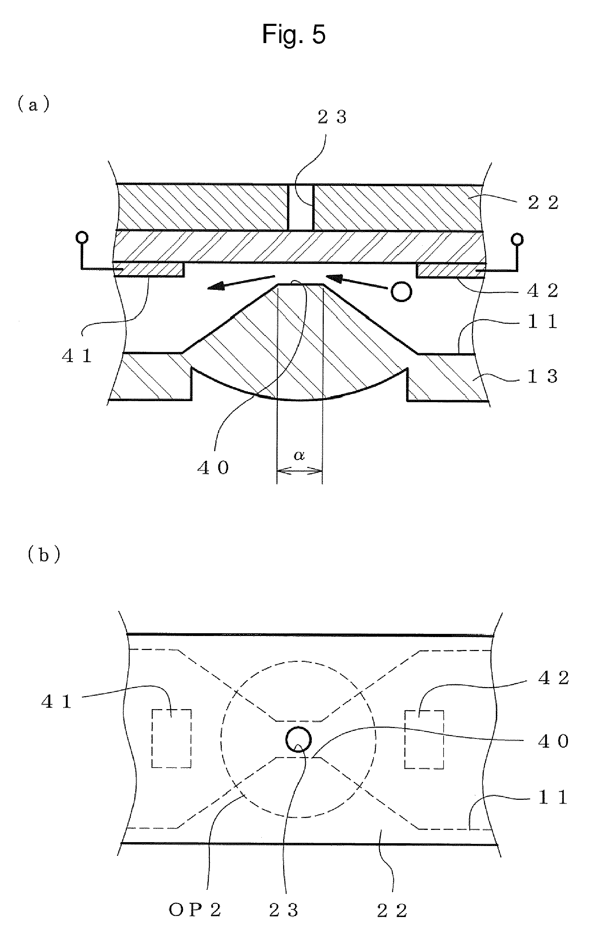

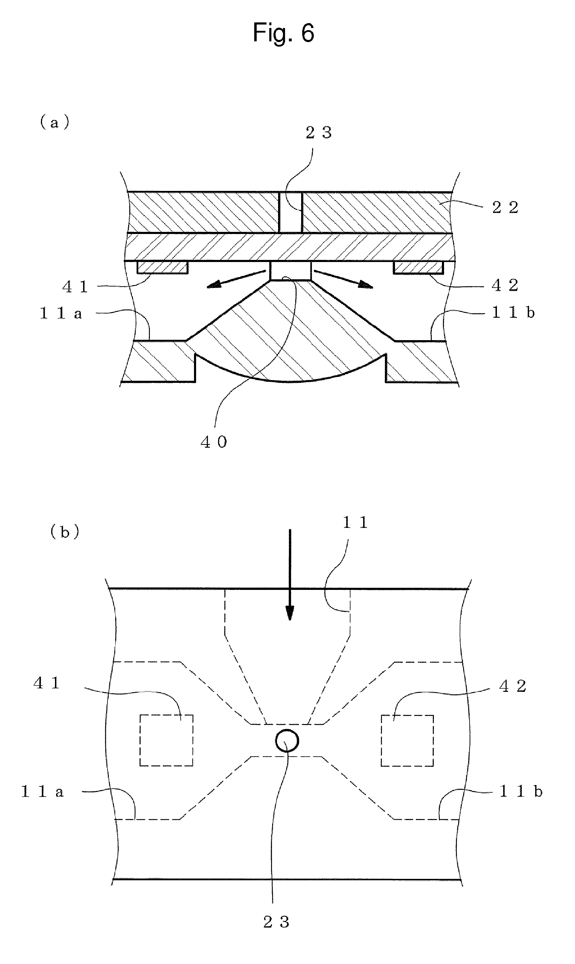

Fig. 6 is a sectional view showing a configuration example of the embodiment of Fig. 5, in which the flow channel diverges in the aperture for the electrical resistance method, wherein a light source device and a light receiving device are omitted. In Fig. 6(a), the apparatus is cut along the right and left flow directions of the diverged flow channel. Fig. 6(b) is a top view of Fig. 6(a), wherein the outline of the main part hidden by a beam-light-forming-film is shown with a broken line. Fig. 6(b) shows the manner of diverging, in the aperture, of the sample solution coming from the upper part into the right and left flow directions in the Figure.
Fig. 7 shows a modification example of the configuration of the apparatus.
Fig. 8 shows one embodiment of the scattergram obtained by the apparatus.
Fig. 9 is a sectional view of a configuration example of a conventional apparatus for flow cytometry.
Fig. 10 explains the basic principle and an apparatus configuration of the electrical resistance method. Fig. 10(a) is a configuration example of an aperture and the electrodes, and Fig. 10(b) schematically shows voltage variation between the electrodes that appears when a blood cell passes through the aperture.
Fig. 11 is a sectional view of the configuration of a conventional apparatus wherein an aperture and an electrode pair for the electrical resistance method are incorporated into the flow cytometer of Fig. 9.
Fig. 12 is a graph showing the time lag between the measurement results by the electrical resistance method and the measurement results by flow cytometry, using the apparatus shown in Fig. 11.

### DETAILED DESCRIPTION OF THE INVENTION

In the particle analysis apparatus of the present invention, conventional complicated parts for an optical system are not used in the light source side, but a beam light is created by a small light transmitting hole made in an opaque film and the beam light reaches the light irradiation point. By such configuration of the light source side, many parts in the optical system are omitted to enable down-sizing of the whole apparatus and the cost of the parts is also reduced.

In addition, since the optical path of the beam light is determined by the position of the light transmitting hole formed in the opaque film, the site on which a beam light is irradiated in the flow channel can be highly accurately aligned. As a result, the steps for alignment and focusing of the irradiation light by many optical system parts can be omitted, and the assembly cost can also be reduced.

Also for the light detector side, in an embodiment wherein a flow channel is formed on the first surface of the below-mentioned substrate for forming the flow channel, the optical system parts of the light receiving device side can also be omitted by integrally forming a condensing lens on the second surface (backside) of the substrate for forming the flow channel, and the alignment of the optical system becomes unnecessary.

As the light source device of the light irradiation device, a semiconductor laser can be used, and the element of the light receiving device can be photodiode. Since the configuration is free of such complicated parts and adjustment thereof, the flow cell part can be a replaceable compact cartridge disposable for each measurement.

According to the present invention, moreover, a configuration for irradiating a beam light with a small diameter toward a particular point in the micro zone in the flow channel can be obtained easily. Therefore, when only the micro zone in the flow channel has a small cross-sectional area to provide an aperture for performing the electrical resistance method, a beam light can be irradiated in the aperture.

Since a measurement by flow cytometry and a measurement by the electrical resistance method can be performed simultaneously by setting a light irradiation point in the aperture, the time lag of the measurements as shown in Fig. 12 is absent, and the problem of time lag can be resolved essentially.

In the following, the configuration of the apparatus of the present invention is explained in detail, and the production method of the present invention is explained by referring to Examples.

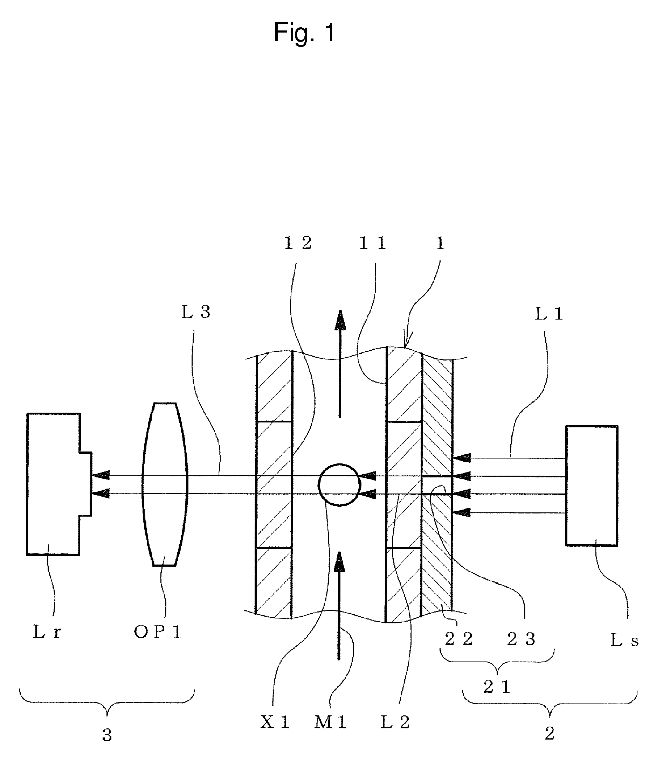

Fig. 1 is a partial sectional view schematically showing one example of the particle analysis apparatus of the present invention. The apparatus has a flow cell part 1, a light irradiation device 2, and a light receiving device 3, so that the particles in a sample solution can be analyzed at least by flow cytometry.

A flow cell part 1 has a flow channel 11 where a sample solution M1 containing particles X1 to be analyzed flows, and has a transparent wall 12 so that the irradiation light can pass through. In the example of Fig. 1, only the part involved in the passage of light is transparent. However, a structure wherein the entire flow cell part is made of the same material is simple and preferable.

A light irradiation device 2 is a device for irradiating an irradiation light L1 as a beam light L2 toward a light irradiation point in a flow channel 11, which is composed of a light source device Ls emitting the irradiation light L1, and a beam-light-forming-film 21 and is free of a lens for focusing the light on the light irradiation point. The light irradiation point is one point designed to be present in a flow channel, onto which the light is focused through a lens in conventional techniques. It is a point on which a particle to be exposed to the light passes (the center of particle X1 in Fig. 1). The beam-light-forming-film 21 is obtained by forming, in an opaque film 22 that does not allow transmission of an irradiation light L1, a light transmitting hole 23 capable of changing the cross sectional shape of the irradiation light L1 to that of a beam light L2. The beam-light-forming-film 21 is placed between a light source device Ls and the light irradiation point, and an irradiation light L1 emitted from a light source device Ls passes through a light transmitting hole 23 in the beam-light-forming-film 21 to become a beam light L2 and is irradiated on the light irradiation point.

The light receiving device 3 is a device for detecting a light L3 (when particle is absent, beam light L2 passes) resulting from the irradiation of a beam light L2 on a particle X1 at the light irradiation point. In this example, optical system parts OP1 for condensing light and a light emitting element Lr are involved.

As mentioned above, since the particle analysis apparatus is free of an optical system such as lens and the like at least on the light source side, the structure is compact, and adjustment of the optical system is not necessary.

The structure of a flow cell part 1 may be similar to that of a conventionally-known flow cell, and may be any as long as a light having a wavelength to be irradiated from the outside can reach at least the light irradiation point in the flow channel, and a transparent wall is contained so that the light formed by the irradiation of the light on the particles flowing in the flow channel can exit to the outside.

The whole shape of the flow cell part may be a circular pipe shape. Since a beam-light-forming-film is connected to the outer surface, the connecting surface thereof is preferably a flat plane and the production thereof including assembly and the like is easy. In addition, the flow cell part as a whole is preferably composed of a transparent material permitting passage of the irradiated light, since the structure can be simplified. Examples of such transparent material include materials of a substrate for forming the flow channel to be explained below.

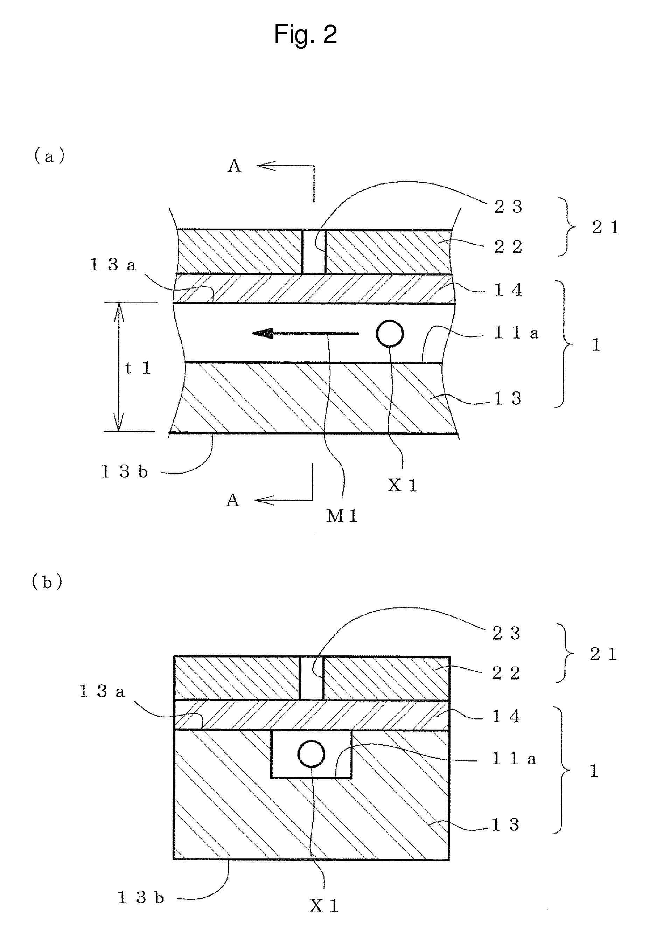

Fig. 2 shows the configuration of the flow cell part suitable for using a beam-light-forming-film.

As shown in this Figure, a groove 11a to be the flow channel 11 is formed on the first surface 13a, which is one main surface of the substrate 13 for forming the flow channel, a transparent covering layer 14 is laminated on the substrate 13 for forming the flow channel, while covering the groove, whereby the groove 11a becomes a conduit flow channel 11 to form a flow cell part 1. An opaque film 22 is laminated on the covering layer 14, and a light transmitting hole 23 aligned with the central part of the flow channel 11 is formed in the film to give a beam-light-forming-film 21. The beam light (not shown) that enters into the groove from the light transmitting hole 23 penetrates the substrate 13 for forming the flow channel and is detected by a light receiving device (not shown) located below in the Figure.

In the example of this Figure, an exclusive covering layer 14 is formed to cover groove 11a to form a flow channel, wherein the covering layer may also be a beam-light-forming-film. In this case, the light transmitting hole is not a simple through-hole but filled with a light transmittable material so that the sample solution in the flow channel will not leak.

In the example of Fig. 2, the shape of the substrate for forming the flow channel is a simple plate. However, various depressions and protrusions, curved surfaces, inclined planes and the like may be formed on the outer surface of the substrate so that it will be suitable for assembly and the like.

The material of the substrate for forming the flow channel may be any as long as it has a mechanical strength as a flow cell and is transparent to allow for penetration of the irradiation light. Examples of the preferable material include resin compositions such as acrylic resin (polymethylmethacrylate (PMMA)), polystyrene (PS), polycarbonate (PC), olefin-based resin (cycloolefin polymer (COP), cycloolefin copolymer (COC)), silicone resin (PDMS) and the like.

The thickness (t1 in Fig. 2) of the substrate for forming the flow channel is not particularly limited, and can be appropriately determined according to the depth of the groove. Since the depth of the flow channel (groove) increases in the parts other than the light irradiation point in flow cytometry and a small flow channel to be the aperture in the electrical resistance method, the thickness t1 is preferably about 0.5 mm - 10 mm, more preferably 1 mm - 3 mm.

When an accessory structure is formed such as lens for condensing light and the like on the second surface 13b of the substrate for forming the flow channel, as mentioned below, a thickness necessary therefor can be added as appropriate.

While a method for processing groove 11a into a substrate 13 for forming the flow channel is not particularly limited, a method including using a forming die and molding, as a component having a groove on the first surface 13a, a resin into a substrate for forming the flow channel is preferable.

As other groove processing methods, a method including later applying a groove processing to a flat plate, a method including further dividing the substrate for forming the flow channel into plural layers (layer for bottom surface, layers for side walls) and building a groove and the like can be mentioned.

The cross sectional shape of the flow channel in the flow cell part (sectional shape when flow channel is perpendicularly cut in the flow direction) is not particularly limited, and it may be a circular shape or a quadrate such as square, rectangle and the like.

As shown in Fig. 2, when a groove is formed in a substrate for forming a flow channel, the cross sectional shape of the groove is preferably a semicircle, a quadrate such as square, rectangle and the like, a U-shape (wherein the bottom line (side to be the bottom of groove) of quadrate is a semicircle) and the like from the aspects of processing. Of these, square is preferable since the flow becomes comparatively uniform and the parallel light is maintained. The square corners may be rounded as necessary.

The cross sectional shape of the flow channel (particularly, the cross sectional shape of a flow channel containing a light irradiation point in flow cytometry and the cross sectional shape of a flow channel to be the aperture in the electrical resistance method) preferably has a size that permits passage of only one of the particles to be analyzed, while being surrounded by the flows of a sample solution and a sheath liquid.

When the particle is a blood cell, the size of the cross sectional shape of the flow channel to be a light irradiation point in flow cytometry and the size of the cross sectional shape of a flow channel to be the aperture in the electrical resistance method are preferably expressed by a diameter of about 20 µm - 200 µm, more preferably 50 µm - 100 µm, when the cross sectional shape is, for example, a circular shape. As shown in Fig. 2, when the flow channel is formed by the grooves in the substrate for forming the flow channel and the cross sectional shape of the groove is square, one side thereof is preferably about 20 µm - 200 µm, more preferably 35 µm - 50 µm.

While the material of the covering layer is not particularly limited, examples of preferable material include acrylic resin (PMMA), polyethylene terephthalate (PET), polycarbonate (PC), olefin-based resin (COC, COP) and the like.

While the thickness of the covering layer is not particularly limited, it is preferably as thin as possible so that it can have the minimum mechanical strength to maintain the flow channel and can avoid being an obstacle for the beam light. It is preferably about 0.05 mm - 1 mm, more preferably 0.1 mm - 0.2 mm.

As a method for laminating a covering layer on a substrate for forming a flow channel, a method including connection via a bonding agent layer or an adhesive layer is preferable. Such bonding agent and adhesive only need to be composed of a stable material that does not exert a harmful effect on the liquid flowing through the flow channel and is resistant to a harmful effect.

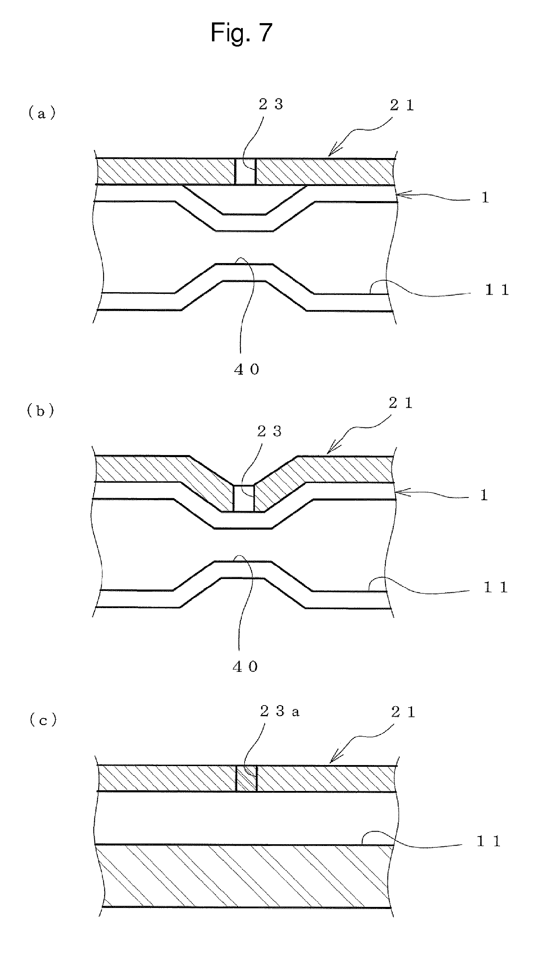

In the embodiments of Fig. 1 and Fig. 2, a beam-light-forming-film is shown as a member belonging to a light irradiation device. As in the modified embodiment shown in Fig. 7(c), a beam-light-forming-film 21 may also be a covering layer, and the beam-light-forming-film may also be a part of a flow cell part. A light transmitting hole 23a in this case needs to be filled with a transparent material so that the liquid in the flow channel will not leak, or only that part needs to be transparent.

In the present invention, a light irradiation device is configured to have a light source device and a beam-light-forming-film. For example, when a light source device is an LED (Light Emitting Diode), a lens for making an irradiation light a partially parallel light may be provided in a resin mold part of the LED.

The light source device is not particularly limited and may be any as long as it can emit light having an appropriate wavelength for the particle to be the object of counting. Examples thereof include a semiconductor laser, a solid-state laser device, a lamp, an LED, a QCL (quantum cascade laser) and the like, with preference given to a semiconductor laser since it shows high light intensity and provides a more compact configuration of the whole apparatus.

The wavelength of the irradiation light varies depending on the particle to be the target of counting and may follow conventional techniques. When the particle is a blood cell, a preferable wavelength is in the range of 500 nm - 700 nm.

The cross sectional shape of the irradiation light may be any as long as it has a size not less than the cross sectional shape of the light transmitting hole in the beam-light-forming-film and can embrace the cross sectional shape of the light transmitting hole.

To suppress spreading of the beam light, the beam-light-forming-film is preferably set closer to the light irradiation point. In a preferable embodiment, therefore, the beam-light-forming-film is closely adhered to the surface of the flow cell part. When a groove is formed on the substrate for forming the flow channel to build a flow channel, an embodiment wherein the covering layer also acts as a beam-light-forming-film is an embodiment that can set the beam-light-forming-film closest to the light irradiation point.

An opaque film 22 to be the base material of the beam-light-forming-film 21 may be an independently-formed film member or an opaque coat formed by coating an outer surface of the flow cell (upper surface of covering layer in the embodiment of Fig. 2) with an opaque paint and the like. Since the production cost is lower, the opaque film is preferably an independently-formed film member.

The opaque film may be any film as long as it does not transmit an irradiation light. In a preferable embodiment, it is a resin film that is colored opaque due to the presence of a pigment or dye that does not transmit irradiation light, since such film permits easy processing for forming the below-mentioned light transmitting hole. Examples of the resin include polyethylene terephthalate (PET), vinyl chloride resin (PVC), polypropylene (PP), polyethylene naphthalate (PEN) and the like.

The pigment and dye to be contained in the resin may be known and, for example, carbon, iron oxide and the like can be mentioned.

To facilitate processing of the light transmitting hole in the below-mentioned production method and suppress reflection of the irradiation light, the color of the pigment and dye is preferably black.

While the thickness of the opaque film is not particularly limited, it is preferably about 20 µm - 500 µm, more preferably 50 µm - 100 µm, to achieve suitable processability and sufficient light impermeability.

When the opaque film is an independently-formed film member, lamination of the opaque film on the surface of a flow cell part by interposing the above-mentioned bonding agent layer or an adhesive layer is performed in a preferable embodiment.

The cross sectional shape of the light transmitting hole to be formed in an opaque film may be any as long as a beam light having a cross sectional shape suitable for flow cytometry can be formed.

The beam light may be not only a line-shaped light having a circular cross sectional shape, but also a flat plate-shaped light after passage of a thin slit. When the beam light is a flat plate-shaped light, the beam light is preferably irradiated such that the band-like thin cross sectional shape of the beam light extends in a direction crossing the flow channel.

The cross sectional shape and the size of the beam light can be appropriately determined according to the particle to be the object of counting. When the particle is a blood cell, the outer diameter in the flow direction of the flow channel of the cross sectional shape of the beam light (when the cross sectional shape is a circular shape, it is a diameter, and when the cross sectional shape is band-like, it is a band width) is preferably about 10 µm - 200 µm, more preferably 50 µm - 100 µm.

Therefore, the above-mentioned cross sectional shape of the beam light can be adopted as the cross sectional shape of the light transmitting hole.

The light transmitting hole may be a through-hole formed in an opaque film (no film material in the through-hole), or a through-hole in which a transparent material is present and only the light can penetrate therethrough. A through-hole is preferable, since it can be formed in an opaque film easily and shows high positioning accuracy.

While the method for forming a light transmitting hole in an opaque film is not particularly limited, it may be perforation processing by irradiation of laser light (local melting with high temperature, local destruction by laser ablation and the liked) and local destruction of dyestuff are preferable methods. In addition, it may be etching a light transmitting hole by using a resist, a method for forming an opaque film except a light transmitting hole on the outer surface of a flow cell by printing process or various deposition methods.

The production method of the present invention provides a preferable method for giving a beam-light-forming-film on a flow cell in the production of the particle analysis apparatus of the present invention.

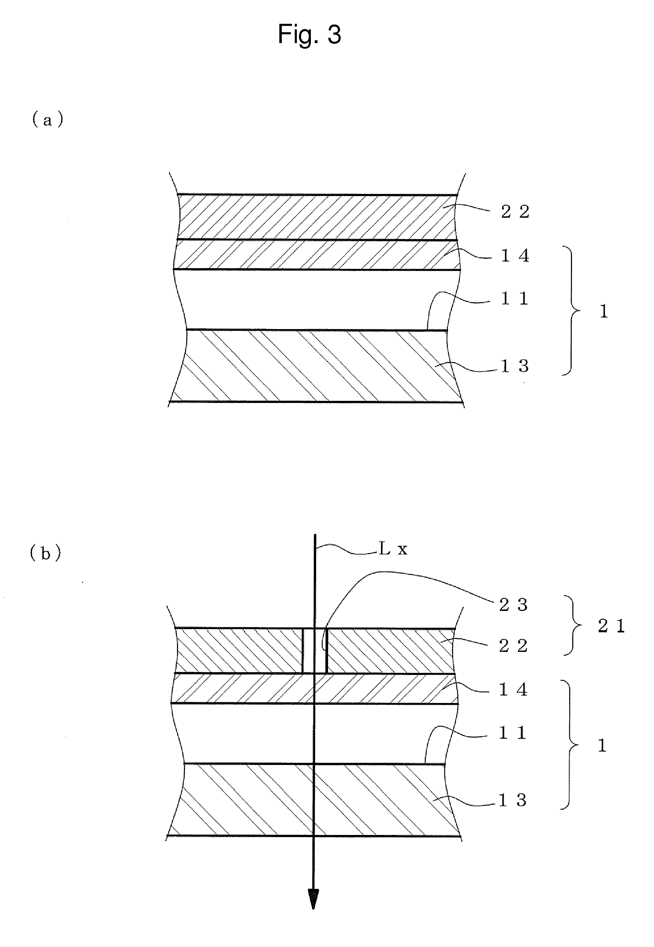

In this production method, as shown in Fig. 3 (a), an opaque film 22 is fixed on the region of a flow cell part 1 where a flow channel 11 containing at least a light irradiation point exists.

Then, as shown in Fig. 3 (b), the opaque film 22 is processed to form a light transmitting hole 23 at a position corresponding to a light irradiation point P, and the opaque film 22 is used as a beam-light-forming-film 21.

In the example of Fig. 3 (b), a fine hole is formed in the opaque film by irradiating a laser light Lx. Since the covering layer 14 therebeneath does not absorb the laser light Lx, a hole is not formed therein.

According to the production method, since the positioning of a laser light to the target is only necessary for forming a light transmitting hole, the processing is easy, the positioning accuracy is high, and highly accurate positioning is not necessary for adhering the opaque film to the flow cell part.

On the contrary, in a method including separately forming a beam-light-forming-film having a light transmitting hole in advance and adhering the film to a flow cell part, the beam-light-forming-film in its entirety needs to be moved to position the already-formed light transmitting hole at the center of the flow channel, and the film needs to be adhered using an adhesive. Since the beam-light-forming-film is thin as mentioned above, such fine adjustment takes time and high accuracy cannot be achieved.

The laser light (laser device) for forming a light transmitting hole in the present production method is not particularly limited, and YAG laser, CO₂ laser, excimer laser, Ar laser and the like are preferable.

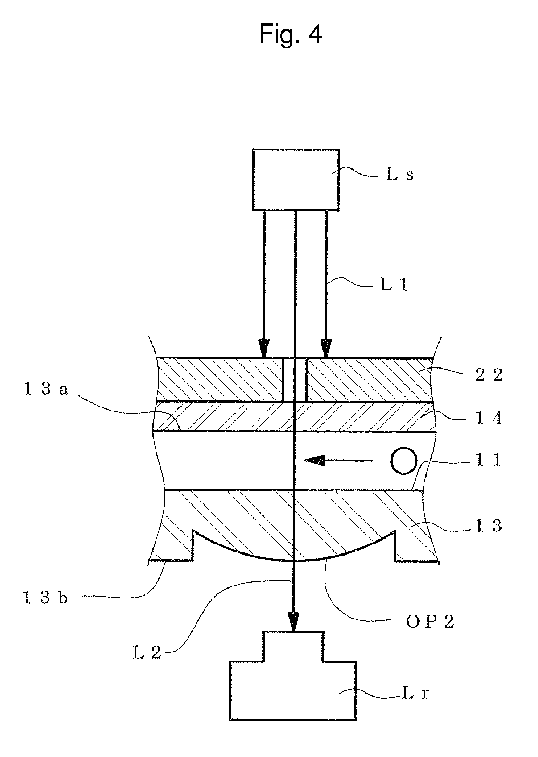

Fig. 4 shows a preferable embodiment for forming a groove on the substrate for forming the flow channel.

In the embodiment shown in this Figure, a curved surface part OP2 that functions as a lens for condensing light is formed integrally with a second surface 13b, which is the other main surface of the substrate 13 for forming the flow channel. The curved surface part OP2 that functions as the lens is provided in the center of the region corresponding to the light irradiation point in the flow channel (groove formed in the first surface 13a), and only needs to be designed to condense the light that has passed through the light irradiation point (light L3 produced by hitting particles on the light irradiation point with a beam light L2 or the beam light L2 that has passed as it is as shown in Fig. 4) toward a light receiving device.

The curved surface part OP2 may be a simple convex lens as shown in Fig. 4, or a lens with a special curved surface like Fresnel lens. It may also be a concave lens when combined with other optical instrument.

While the curved surface part OP2 that functions as the lens may be formed by after processing of the second surface 13b of the substrate 13 for forming the flow channel, a method including resin molding a substrate for forming the flow channel as a component having a curved surface on the second surface 13b, like the groove on the first surface, by using a forming die is preferable.

Fig. 5 shows a more preferable embodiment of the apparatus of the present invention.

In the embodiment of Fig. 5, a configuration capable of performing the analysis of particles by the electrical resistance method simultaneously with the measurement by flow cytometry is further imparted in addition to the configuration shown in Fig. 4. As shown in Fig. 5, the cross-sectional area of the flow channel (groove) 11 decreases toward the micro zone such that the micro zone (length α) containing a light irradiation point in the flow channel 11 is an aperture 40 for performing the electrical resistance method. In addition, electrodes 41, 42 for performing the electrical resistance method are provided on the upstream side and the downstream side in the flow channel in the Figure, with the aperture 40 interposed between them.

In the embodiment in this Figure, the beam-light-forming-film for irradiating a beam on the light irradiation point and the curved surface part OP2 that functions as the lens formed on the second surface 13b of the substrate 13 for forming the flow channel are the same as in the embodiment explained in Fig. 4.

By this configuration, it is possible to perform an optical analysis by light irradiation for flow cytometry in one micro zone and perform an electric analysis by the electrical resistance method at the same site by using the micro zone as an aperture. Therefore, a temporal deviation (time lag) of the measurement results, as shown in Fig. 12, is essentially absent.

The micro zone only needs to have a zone having a length α and capable of functioning as an aperture for performing the electrical resistance method. As explained for the flow channel, the cross sectional shape of a micro zone preferable as the aperture preferably has a size permitting passage of only one particle while being surrounded by the flow of a sample solution or a sheath liquid.

While the length α of a micro zone preferable as an aperture for performing the electrical resistance method varies depending on the particle, when the particle is a blood cells, it is within the range of about 20 µm - 200 µm, more preferably 40 µm - 100 µm.

The flow channel (groove) for performing the electrical resistance method gradually increases in the depth direction and the width direction, as shown in Fig. 5(a), (b), as it gets farther from the micro zone to be the aperture. As for such increase and decrease per se of the flow channel, the prior art may be referred to.

Fig. 6 shows a special configuration example of the embodiment shown in Fig. 5.

In the embodiment of Fig. 6, the flow channel diverges in the micro zone (aperture 40) to more preferably perform the electrical resistance method with the configuration shown in Fig. 5. As shown in Fig. 6(b), a sample solution that has flown from the flow channel 11 in the upper part enters the micro zone (part where light transmitting hole 23 is formed), and flows out from flow channels 11a, 11b in the left and right sides of the Figure. Such diverge of the flow channel may be formed on the outlet port at the downstream side of the micro zone (aperture). While the electrodes 41, 42 are provided such that the micro zone is placed between them, since the flow channel diverges, both electrodes are positioned on the downstream side of the micro zone. Such diverge of the flow channel and the arrangement of the electrodes prevent passage of babbles generated on the electrodes through the aperture, whereby an effect of decreased noise of the babble can be obtained.

Fig. 7 shows modification examples of the configurations shown in Fig. 4 - Fig. 6.

In the example of Fig. 7(a), the outer shape of a flow cell part 1 is tapered to form an aperture 40, and a gap is present between a flat plate-shaped beam-light-forming-film 21 and a flow cell part 1.

In the example of Fig. 7(b), the outer shape of a flow cell part 1 is tapered to form an aperture 40, and a beam-light-forming-film 21 is in close contact with the outer shape of the flow cell part 1, leaving no gap between them.

In the example of Fig. 7(c), a beam-light-forming-film 21 is a part of a flow cell part 1.

The particle to be counted in the present invention is not particularly limited and may be, for example, fine particle, liposome and the like encapsulating a medical agent and the like. When blood cells such as red blood cells, leukocytes, platelets and the like are to be counted, the usefulness of the apparatus of the present invention is remarkable since it can be miniaturized to a size that fits in a palm.

Moreover, it is possible to produce a part of the flow channel containing a light irradiation point for performing flow cytometry (additionally, a part of the flow channel containing an aperture for the electrical resistance method) as a cartridge type replaceable (disposable) from the apparatus body. Also in this point, the present invention is useful as a blood cell counting apparatus.

When the blood cells are to be counted, the prior art may be referred to as regards sample solution and sheath liquid, the intensity and light sensitivity of irradiation light for flow cytometry, applied voltage for the electrical resistance method, graphing of particle count results by flow cytometry (making frequency distribution graph or scattergram), and further, graphing, analysis method, data processing method and the like of particle count results by the electrical resistance method to be combined.

One embodiment using the apparatus for the measurement for classification of leukocytes is shown below.

A sample solution is prepared by subjecting the whole blood to hemolysis, dilution and staining, and the individual blood cell is measured by the apparatus for the absorbance by flow cytometry and volume by the electrical resistance method.

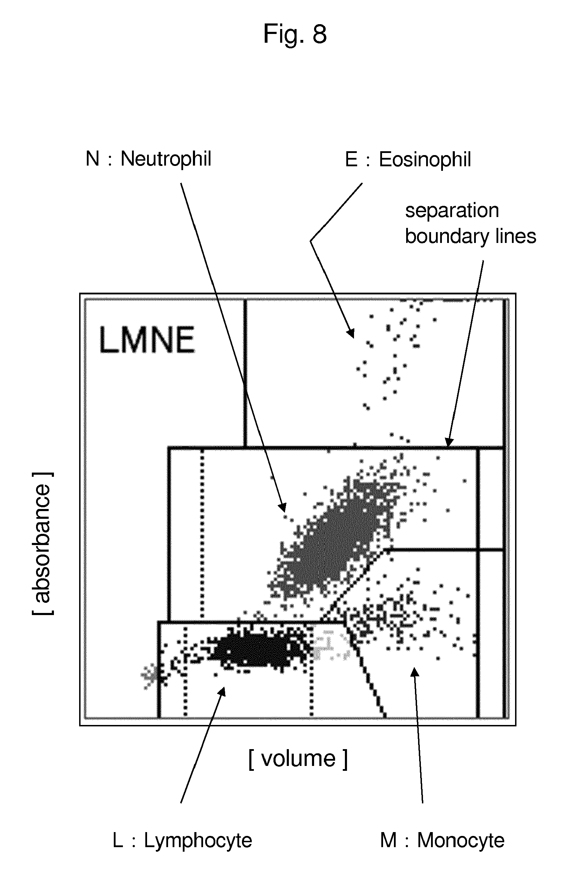

The obtained measurement data (volume, absorbance) of each blood cell are plotted on the X-Y plane consisting of X-axis (horizontal axis corresponding to volume) and Y-axis (vertical axis corresponding to absorbance) to give a scattergram as shown in Fig. 8. The obtained scattergram is called an LMNE matrix, which clearly shows the distribution of four kinds of leukocytes, i.e., lymphocyte (L), monocyte (M), neutrophil (N) and eosinophil (E).

Since the apparatus of the present invention enables measurement of the absorbance by flow cytometry and volume by the electrical resistance method at the same position by a simple configuration, the graph has high reliability with a fewer errors caused by the time lag, even though the apparatus is compact.

According to the present invention, large-scale structures of the optical system, which are problematic for conventional apparatuses for flow cytometry, can be omitted, the apparatuses can be down-sized, as well as adjustment and processing have been simplified. In addition, since flow cytometry and electrical resistance method can be performed at the same position, the problem of time lag has been resolved. As a result, an apparatus particularly preferable for the classification and counting of blood cells can be provided.

## Claims

1. A particle analysis apparatus having a configuration adapted to analyze particles in a sample solution at least by flow cytometry, the apparatus comprising:
a flow cell part having a flow channel where the sample solution containing the particles to be analyzed flows;
a light irradiation device for irradiating an irradiation light as a beam light to a light irradiation point in the flow channel; and
a light receiving device for detecting the light resulting from the irradiation of the beam light to the light irradiation point;
wherein
said light irradiation device comprises:
a light source device for emitting the irradiation light; and
a beam-light-forming-film composed of an opaque film prohibiting transmission of the irradiation light, and a light transmission through hole capable of changing the cross sectional shape of the irradiation light to a cross sectional shape of the beam light,
and wherein
the light irradiated from the light source device passes through the light transmitting hole in the beam-light-forming-film to form the beam light to be irradiated on the light irradiation point.

2. The particle analysis apparatus according to claim 1, wherein the flow cell part and the flow channel are configured by a groove formed on a first surface, which is one main surface of the substrate for forming the flow channel, and a covering layer laminated on said substrate to cover and provide the groove as a conduit flow channel, wherein
the covering layer is transparent, and said beam-light-forming-film is further laminated thereon, or the covering layer is also said beam-light-forming-film.

3. The particle analysis apparatus according to claim 1 or 2, further comprising a second surface, which is the other main surface of the substrate for forming the flow channel, integrally formed with a curved surface part that functions as a lens for condensing the light, that has passed the light irradiation point, toward the light receiving device, said curved surface part having, as the center, a region corresponding to the light irradiation point in the flow channel in the first surface.

4. The particle analysis apparatus according to any one of claims 1 to 3, having a configuration also enabling analysis of particles in a sample solution by an electrical resistance method, wherein
said flow channel comprises a micro zone containing the light irradiation point and having a decreased cross-sectional area such that the micro zone becomes an aperture for the electrical resistance method, and electrodes for performing the electrical resistance method, which are provided at positions placing the aperture between them, which apparatus enabling the flow cytometry and the electrical resistance method to be performed in a single micro zone.

5. The particle analysis apparatus according to any one of claims 1 to 4, wherein the flow channel diverges in said micro zone or at an outlet port on the downstream side of the micro zone.

6. A method of producing the particle analysis apparatus according to any one of claims 1 to 5, comprising the steps of:
fixing an opaque film to the flow cell part having the flow channel so as to cover a region including the flow channel containing at least the light irradiation point, and
processing the opaque film to form a light transmitting hole at a position corresponding to the light irradiation point to provide a beam-light-forming-film.
